# EUROPÄISCHE PATENTSCHRIFT

(11) **EP 2 238 946 B1**
(45) Veröffentlichungstag und Bekanntmachung des Hinweises auf die Patenterteilung: **08.02.2012**
(21) Anmeldenummer: 10003315.8
(22) Anmeldetag: 27.03.2010
(51) Int. Cl.: A61F 2/18

(54) **Längenvariable Gehörknöchelchenprothese**
Ossicular prosthetics with variable length
Prothèse d'osselets d'oreille à longueur variable

(30) Priorität: 10.04.2009 DE 102009016468
(43) Veröffentlichungstag der Anmeldung: 13.10.2010
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE); Gamer, Walter, 76297 Stutensee (DE)
(74) Vertreter: Kohler Schmid Möbus

(56) Entgegenhaltungen:
- WO-A2-2008/027862

## Beschreibung

Die Erfindung betrifft eine Gehörknöchelchenprothese, die mindestens ein Glied oder Teile eines Gliedes der menschlichen Gehörknöchelchenkette ersetzt oder überbrückt, wobei die Gehörknöchelchenprothese an ihrem einen Ende ein erstes Befestigungselement zur mechanischen Verbindung mit dem Trommelfell oder einem Glied der Gehörknöchelchenkette, insbesondere mit dem Ambossfortsatz oder mit dem Hammergriff, und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes, längliches Verbindungselement aufweist, welches eine Verstellvorrichtung zur Einstellung der axialen Länge der Gehörknöchelchenprothese in Achsrichtung des länglichen Verbindungselements umfasst, wobei das erste Befestigungselement mit einem Ende und das zweite Befestigungselement mit dem axial entgegen gesetzten anderen Ende des Verbindungselements mechanisch starr verbunden ist.

Eine derartige Vorrichtung ist bekannt aus der DE 20 2007 012 217 U1.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehör-knöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung bzw. Signalübertragung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Gehörknöchelchenprothese entweder eine ausreichend große Auswahl von Prothesen unterschiedlicher axialer Länge bereitgehalten werden oder die verwendeten Gehörknöchelchenprothesen müssen während der Operation ausgehend von einer maximalen Ausgangslänge auf die erforderliche axiale Endlänge gebracht werden können.

Eine mittels Klemmwirkung längenverstellbare Anordnung zum mechanischen Ankoppeln des Treibers eines aktiven Hörgeräts an eine Ankoppelstelle der Gehörknöchelchenkette ist in der DE 199 48 375 A1 beschrieben.

In der WO 92/18066 A1 ist eine selbstanpassende passive Gehörknöchelchenprothese beschrieben, die einen komplizierten und in der Herstellung sehr aufwändigen Federmechanismus in der Verbindung zwischen dem ersten und dem zweiten Befestigungselement aufweist, welcher eine ständige Änderung der axialen Länge der Prothese je nach relativer Lage der Befestigungspunkte im Mittelohr bewirkt. Eine reproduzierbar exakte, feste Längeneinstellung der Prothese, die auch nach dem operativen Einsetzen derselben ins Mittelohr erhalten bleibt, ist damit nicht möglich. Zudem erfordert die bekannte Prothese wegen ihres sehr speziellen mechanischen und geometrischen Aufbaus einen erheblichen Platzbedarf im Mittelohr, so dass sie in vielen Fällen aufgrund der individuellen Gegebenheiten beim Patienten gar nicht einsetzbar ist. Außerdem wird Konstruktionsbedingt nach dem Einsetzen ein nicht unerheblicher permanenter Druck zwischen den beiden Befestigungspunkten im Mittelohr aufgebaut, was einer Heilung nach der Operation nicht gerade förderlich ist und auf Dauer häufig zu postoperativen Komplikationen führt.

Eine passive Gehörknöchelchenprothese mit während der Operation in bestimmten Grenzen variierbarer axialer Länge ist in der DE 39 01 796 A1 beschrieben. Dabei wird die Längenänderung durch ein Verbiegen des als dünner aus Golddraht gefertigten Verbindungselements erreicht, was allerdings einerseits umständlich in der Handhabung, andererseits ziemlich ungenau ist, so dass damit keine exakte Einstellung der gewünschten axialen Länge der Gehörknöchelchenprothese erreicht werden kann. Außerdem ist das Ergebnis bei dieser Technik nicht immer reproduzierbar und es kann sogar vorkommen, dass sich nach dem Verbiegen des Verbindungselements die eingestellte axiale Länge der Gehörknöchelchenprothese durch ein Zurückfedern des Verbindungselements wieder ändert.

Die EP 0 998 884 A2 beschreibt eine passive Gehörknöchelchenprothese, bei welcher das als länglicher Schaft ausgebildete erste Verbindungselement durch eine Durchgangsbohrung des als Kopfplatte ausgebildeten ersten Befestigungselements hindurch gesteckt wird, bis eine gewünschte Schaftlänge zwischen dem ersten und dem zweiten Befestigungselement erreicht ist. Sodann wird der Schaft in dieser Position durch Verengen der Durchgangsbohrung in der Kopfplatte fixiert und der über die Kopfplatte hinaus überstehende Teil des Schaftes abgelängt. So erhält man auf einfache Weise eine Prothese mit der jeweils gewünschten bzw. erforderlichen, insbesondere nach der Operation exakt gleich bleibenden axialen Länge.

Aus der DE 10 2005 010 705 B3 ist eine Gehörknöchelchenprothese bekannt, bei der eine intraoperative Variabilität der Prothesenlänge dadurch erreicht wird, dass das längliche Verbindungselement in Form einer Kugelkette ausgebildet ist. Diese wird während der Operation mit einer bestimmten Anzahl von Kugeln durch eine Aufnahmeöffnung des ersten Befestigungselements hindurch gesteckt. Danach wird die Kugelkette in der Aufnahmeöffnung des Befestigungselements durch beiderseits der Kugelkette angreifende federnde Stegelemente fixiert und der durch die Aufnahmeöffnung ragende, überstehende Teil der Kugelkette abgezwickt, so dass die Prothese am Ende schließlich genau die gewünschte axiale Länge aufweist. In ähnlicher Weise wird eine Längenvariabilität auch bei einer Gehörknöchelchenprothesen nach der DE 20 2005 015 944 U1 erreicht, wobei hier wiederum eine abzwickbare Kugelkette als Verbindungselement verwendet wird, jedoch die Aufnahme im ersten Befestigungselement anders gestaltet ist.

Eine weitere passive Gehörknöchelchenprothese mit intraoperativ veränderbarer axialer Länge ist in der US-A 3,710,399 beschrieben. Hier wird ein zweigeteiltes Verbindungselement zwischen den beiden Befestigungselementen verwendet, das zwei parallel verlaufende gerade Drahtstücke umfasst, von denen das eine vom ersten und das andere vom zweiten Befestigungselement wegragt. Die beiden Drahtstücke können entweder mittels Drahtschlingen an ihren Enden mit dem jeweils andern Drahtstück verbunden oder in eine Art Verbindungsmuffe mit zwei parallelen Längsbohrungen für die beiden Drahtstücke gesteckt werden. Im ersteren Fall ist jedoch die Fixierungsposition und damit die relative Lage der beiden Drahtstücke nur sehr ungenau einzustellen, so dass eine exakte und reproduzierbare Längeneinstellung der Prothese nicht möglich ist. Im zweiten Fall kann es nach dem Einstecken der Drahtstücke in die Verbindungsmuffe leicht zu Verkippungen, Verknickungen oder Verschiebungen der relativen Lagen der Drahtstücke zueinander kommen, wodurch ebenfalls eine genaue Einstellung der axialen Prothesenlänge erschwert bzw. unmöglich gemacht wird.

Wiederum eine andere Technik der Längeneinstellung wird bei einer passiven Gehörknöchelchenprothese angewendet, wie sie aus der DE 10 2005 027 215 A1 bekannt ist. Diese Prothese zielt ausschließlich auf die Situation einer Steigbügel-Operation ab, so dass immer ein kolbenförmiges Piston als zweites Befestigungselement vorgesehen ist. In diesem Piston ist ein Aufnahmemechanismus angeordnet, in welche das schaftförmige Verbindungselement in axialer Richtung eingeschoben werden soll. Von dem Verbindungselement radial abgespreizte Blattfedern sollen dann in einer gewünschten relativen Position zwischen Verbindungselement und zweitem Befestigungselement eine Arretierung bewirken. Abgesehen davon, dass eine exakt reproduzierbare Einstellung einer gewünschten axialen Länge der Prothese damit nicht immer garantiert sein dürfte, ist der Anwendungsbereich dieser Gehörknöchelchenprothese lediglich auf Steigbügel-Operationen beschränkt, bei der über das Piston eine unmittelbare Verbindung zum Innenohr hergestellt wird. Soll jedoch als zweites Befestigungsteil beispielsweise eine Glocke, ein Stempel, ein Clip oder ein flacher Schuh für eine Verbindung mit einem anderen Teil der Gehörknöchelchenkette verwendet werden, ist diese bekannte Prothese nicht verwendbar. Wenn man nämlich einen entsprechenden Aufnahmemechanismus im zweiten Befestigungsteil unterbringen will, so funktioniert dies schon aus geometrischen Gründen nur in einem Kolben, aber niemals in einer Glocke, einem flachen Schuh oder gar in einem Clip.

Einen ebenfalls stark eingeschränkten Anwendungsbereich deckt die in der DE 297 22 084 U1 beschriebene längenvariable Gehörknöchelchenprothese ab, die anstelle eines schaftförmigen Verbindungselements drei stativartig abknickbare Stegelemente aufweist, welche einenends in einen glockenförmigen oder stempelförmigen Körper zur Befestigung am Steigbügel und anderenends in eine Kopfplatte zur Anlage am Trommelfell münden. Diese Konstruktion kann ausschließlich in Verbindung mit einem plattenartigen Befestigungselement, also lediglich bei Ankopplung am Trommelfell eingesetzt werden. Nachteilig ist bei dieser Prothese außerdem, dass kein definierter Schaft als Verbindungselement zwischen den beiden Befestigungselementen vorhanden ist, so dass es bei nicht völlig exakter axialer Krafteinleitung leicht zu einem Ausweichen bzw. Ausknicken quer zur Längsachse der Prothese kommen kann.

Die in der US-A 5,554,188 beschriebene Gehörknöchelchenprothese umfasst wiederum ein als zweigeteilter Schaft aufgebautes Verbindungselement, bei dem der erste, stangenförmige Abschnitt in eine Aufnahmebohrung des als Aufnahmeteil ausgebildeten zweiten Abschnitts einführbar und axial in der Bohrung verschiebbar ist. Um eine gewünschte axiale Länge der Prothese zu erhalten, wird der stangenförmige erste Abschnitt ausgehend von einer maximalen Ausgangslänge auf eine geeignete Endlänge abgeschnitten und bis auf Anschlag in den zweiten Abschnitt eingeschoben. Durch eine entsprechende Gestaltung des lichten Durchmessers der Aufnahmebohrung relativ zum Außendurchmesser des ersten Abschnitts soll nun eine friktionale Verklemmung von erstem und zweitem Abschnitt eine gewisse Fixierung der Prothesenlänge bewirken, wobei die eigentliche Fixierung dadurch erreicht wird, dass sich die gegeneinander beweglichen Teile der Prothese nach der operativen Einführung ins Mittelohr aufgrund ihres jeweiligen Anschlages an den beiden Befestigungspunkten nicht allzu weit voneinander entfernen können. Eine exakt gleich bleibende Prothesenlänge kann damit allerdings auf Dauer nicht sichergestellt werden.

Bei der in der US 2003/0097178 A1 beschriebenen passiven Gehörknöchelchenprothese weist darüber hinaus das Aufnahmeteil einen in Richtung auf das Einschiebeteil hin offenen Hohlraum auf, der sich in axialer Richtung des Verbindungselements erstreckt, das Verbindungselement ist in axialer Richtung zwischen dem Aufnahmeteil und dem Einschiebeteil Längen variabel gestaltet, und die Festlegung der konkreten axialen Länge des Verbindungselements einer individuellen Gehörknöchelchenprothese erfolgt durch Verklemmen des Einschiebeteils mit dem Aufnahmeteil in einer gewünschten relativen koaxialen Einschiebeposition. Damit könnte im Prinzip eine gewünschte, definierte Länge der Prothese auch schon vor einem Einklemmen zwischen den beiden Befestigungspunkten hergestellt werden, wobei diese Länge auch nach Abschluss der Operation, etwa nach dem Durchstecken eines als Piston ausgebildeten zweiten Befestigungselements durch eine perforierte Steigbügel-Fußplatte, fix beibehalten wird.

Aus der eingangs zitierten DE 20 2007 012 217 U1 schließlich ist eine gattungsgemäße Gehörknöchelchenprothese bekannt, bei welcher die Klemmkraft zwischen dem Aufnahmeteil und dem Einschiebeteil im verklemmten Zustand erheblich größer gewählt wird als die maximalen, natürlicherweise im Mittelohr im Bereich der Gehörknöchelchen auftretenden äußeren Kräfte. Dies ermöglicht eine Längenvariabilität der passiven Gehörknöchelchenprothese "in situ" bzw. intraoperativ, wobei weder größere Sortimente von Prothesen unterschiedlicher Längen während jeder Operation bereitgehalten werden müssen. Außerdem ist die Einstellung der jeweils gewünschten individuellen Prothesenlänge und damit deren Handhabung besonders einfach. Nachträgliche postoperative unerwünschte Längen- und/oder Lageänderungen der Prothese werden durch die vorgeschriebene Wahl der Klemmkraft sicher vermieden. Zudem ist diese bekannte Gehörknöchelchenprothese universell bei allen denkbaren Arten von Ankoppelungen im Mittelohrraum einsetzbar und nicht auf eine bestimmte Klasse von Operationen beschränkt, während beispielsweise die Prothese gemäß der oben zitierten DE 10 2005 027 215 A1 ausschließlich in der Situation einer Steigbügel-Operation verwendet werden kann. Erkauft werden diese Vorteile allerdings durch einen relativ komplizierten mechanischen Aufbau der Verstellvorrichtung im Verbindungselement der Prothese, der natürlich erheblichen Fertigungsaufwand und damit höhere Herstellungskosten verursacht.

Eine Gehörknöchelchenprothese gemäß dem Oberbegriff von anspruch 1 ist aus der WO 2008/027862 A2 bekant

Aufgabe der vorliegenden Erfindung ist es demgegenüber, eine gattungsgemäße längenvariable Mittelohrprothese der eingangs beschriebenen Art mit möglichst einfachen technischen Mitteln unaufwändig und kostengünstig dahin gehend zu verbessern, dass einerseits die Anzahl der intraoperativ bereit zuhaltenden unterschiedlichen Prothesen ganz erheblich reduziert werden kann, ohne dabei die Möglichkeit zur optimalen Adaption der Prothese im konkreten Einzelfall zu verlieren, andererseits aber die komplexe Konstruktion der Verstellvorrichtung der aus der DE 10 2005 027 215 A1 bekannten Gehörknöchelchenprothese durch einen wesentlich einfacheren mechanischen Aufbau umgangen wird.

Erfindungsgemäß wird diese Aufgabe auf ebenso überraschend einfache wie wirkungsvolle Weise dadurch gelöst, dass die Verstellvorrichtung mindestens zwei symmetrisch zur Längsachse des Verbindungselements verlaufende, in Achsrichtung streckbare und/oder stauchbare Teilstränge umfasst, die dauerhaft bleibend plastisch verformbar und zumindest vor ihrem Verformen quer zur Längsachse in mehreren Schlaufen gefaltet sind.

Durch die plastische Verformbarkeit aufgrund der erfindungsgemäß gestalteten Geometrie der Verstellvorrichtung, die nach der Verformung eine dauerhaft bleibende Prothesenlänge sicherstellt, wird einerseits eine Längenverstellbarkeit der Gehörknöchelchenprothese in sehr weiten Grenzen erhalten, so dass in der Regel für jeden Prothesentyp mit unterschiedlicher Ausgestaltung der Befestigungselemente jeweils nur eine einzige Ausführungsform der erfindungsgemäßen Gehörknöchelchenprothese bereitgehalten werden muss, mit welcher dann jede erforderliche Prothesenlänge individuell beim Patienten intraoperativ erzeugbar ist. Andererseits senkt der überaus einfache erfindungsgemäße Aufbau der Verstellvorrichtung die Herstellungskosten erheblich. Außerdem ist auch die Handhabung für den Operateur bei der individuellen Längenanpassung einfacher als bei den meisten bekannten Prothesen.

Ganz besonders bevorzugt sind Ausführungsformen der erfindungsgemäßen Gehörknöchelchenprothese, bei denen mindestens ein, vorzugsweise mehrere quer zur Längsachse verlaufende Verbindungsstege vorgesehen sind, welche jeweils eine Schlaufe eines Teilstrangs mit einer Schlaufe eines parallelen eines Teilstrangs verbinden. Dadurch werden beim Auseinanderziehen oder Stauchen der Verstellvorrichtung die parallelen Teilstränge an bestimmten Stellen jeweils in einer exakt definierten Distanz zueinander gehalten, so dass die Verstellvorrichtung nach ihrer plastischen Verformung eine genau vorgegebbare Geometrie aufweist.

Um eine möglichst große Bandbreite der erreichbaren axialen Längen einer erfindungsgemäßen Gehörknöchelchenprothese zu erhalten, umfasst bei vorteilhaften Ausführungsformen jeder Teilstrang mindestens drei Schlaufen.

Eine Klasse von Ausführungsformen der erfindungsgemäßen Gehörknöchelchenprothese zeichnet sich dadurch aus, dass die Schlaufen der Verstellvorrichtung im angelieferten Zustand der Prothese eng zusammengefaltet sind, zur Einstellung einer gewünschten axialen Länge der Prothese in Richtung der Längsachse des länglichen Verbindungselements von einem Operateur auseinander gezogen werden können und nach der Implantation der Prothese ins Mittelohr des Patienten in diesem auseinander gezogenen Zustand plastisch verformt verharren.

Bei einer dazu alternativen Klasse von Ausführungsformen sind die Schlaufen der Verstellvorrichtung im angelieferten Zustand der Gehörknöchelchenprothese auseinander gezogen und können zur Einstellung einer gewünschten axialen Länge der Prothese in Richtung der Längsachse des länglichen Verbindungselements von einem Operateur zusammen gestaucht werden. Nach der Implantation der Prothese ins Mittelohr des Patienten verharren sie dann plastisch verformt in diesem zusammengestauchten Zustand.

In der Praxis bewähren sich Ausführungsform der erfindungsgemäßen Gehörknöchelchenprothese bei denen die Schlaufen der Teilstränge der Verstellvorrichtung Schlangenförmig, Mäanderförmig bzw. Ziehharmonika-artig gefaltet sind.

Um ein gleichmäßiges, definiertes Dehnen bzw. Stauchen der Verstellvorrichtung beim plastischen Verformen zu erzielen, weisen bei einer Klasse von Ausführungsformen die Schlaufen der Verstellvorrichtung quer zur Längsachse des länglichen Verbindungselements jeweils die gleiche maximale Ausdehnung auf.

Bei einer alternativen Klasse von Ausführungsformen weisen axial gegenüberliegende Schlaufenpaare der Verstellvorrichtung quer zur Längsachse des länglichen Verbindungselements unterschiedliche, insbesondere von einem axialen Ende der Verstellvorrichtung zum anderen axialen Ende hin stetig zunehmende oder abnehmende maximale Ausdehnungen auf. Damit kann eine bestimmte Reihenfolge vorgegeben werden, in der die einzelnen Schlaufen sich beim Dehnen bzw. Stauchen der Verstellvorrichtung verformen. Diejenige Schlaufe mit der größten maximalen Ausdehnung wird im Hinblick auf ihre plastische Verformbarkeit in der Regel die weichste sein und sich daher bei Krafteinwirkung als erste verformen.

Bei einer besonders einfachen und kompakten Klasse von Ausführungsformen der erfindungsgemäßen Gehörknöchelchenprothese bildet die Verstellvorrichtung selbst schon das längliche Verbindungselement.

Bei einer dazu alternativen Klasse von Ausführungsformen ist das längliche Verbindungselement - wie üblich - als Schaft gestaltet.

Bevorzugt sind Weiterbildungen dieser Ausführungsformen, bei denen die Verstellvorrichtung in den Schaft integriert und an beiden axialen Enden der Verstellvorrichtung ein Verbindungsstück zum ersten Befestigungselement bzw. zum zweiten Befestigungselement angeordnet ist, was der Prothese in axialer Richtung eine gewisse Formstabilität verleiht.

Ganz besonders vorteilhaft sind Ausführungsformen der erfindungsgemäßen Gehörknöchelchenprothese, bei denen an beiden axialen Enden der Verstellvorrichtung, vorzugsweise mit axialem Abstand zum ersten Befestigungselement bzw. zum zweiten Befestigungselement, jeweils eine Eingriffseinrichtung vorgesehen ist, an welcher mit einem Bedingungsinstrument, beispielsweise einer Pinzette oder Zange, jeweils ein Kraft- oder Formschluss hergestellt werden kann, um die Verstellvorrichtung durch Krafteinwirkung in Richtung der Längsachse des Verbindungselements auseinander zu ziehen oder zu stauchen.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In einigen Ausführungsformen der Erfindung wird das erste Befestigungselement eine zur Anlage am Trommelfell ausgebildete Kopfplatte umfassen. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über eine Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen einer Kopfplatte und dem Verbindungselement kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

In der Regel wird bei der erfindungsgemäßen GehörknöchelchenProthese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Ausführungsform der Erfindung im länglichen Verbindungselement mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Möglich sind Ausführungsformen der Erfindung, bei denen die Prothese oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der erfindungsgemäßen Gehörknöchelchenprothese grundsätzlich eine wachstumsfördernde Beschichtung, ein direkt ins Innenohr führendes, etwa in Form eines Kolbens ausgebildetes Befestigungselement hingegen eine wachstumshemmende Beschichtung aufweisen.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehör-knöchelchenprothese erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

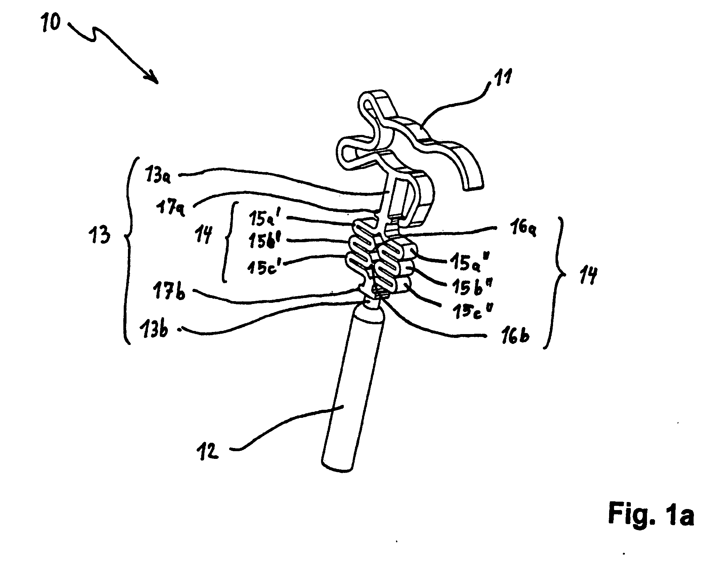

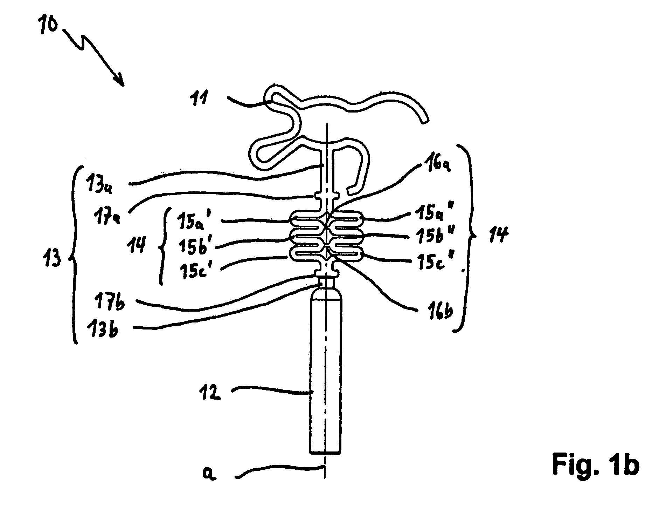

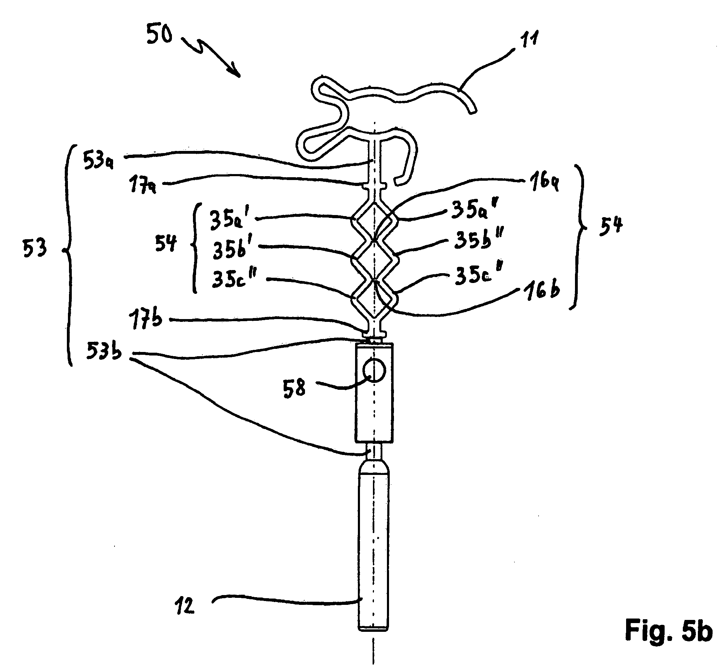

Die Figuren 1a, 1b bis 5a, 5b der Zeichnung sind in 2er-Gruppen unterteilt. Die "a"-Figuren enthalten jeweils eine schematische räumliche Darstellung einer Ausführungsform der erfindungsgemäßen Gehörknöchelchenprothese, die "b"-Figuren einen zur entsprechenden "a"-Figur gehörigen vertikalen Längsschnitt durch die Ausführungsform. Im Übrigen sind in der Zeichnung Elemente mit gleichem Aufbau und/oder gleicher Funktion jeweils mit derselben Bezugsziffer bezeichnet.

Im Einzelnen zeigen:
- Fign. 1a,b: eine Ausführungsform der erfindungsgemäßen Gehör- knöchelchenprothese mit einem klammerförmigen ersten Befestigungselement, einer Verstellvorrichtung mit eng zusammengefalteten Schlaufen von jeweils gleicher Querausdehnung sowie einem kolbenförmigen zweiten Befestigungselement;
- Fign. 2a,b: eine Ausführungsform mit einem als Trommelfell-Kopfplatte gestalteten, ringförmig aufgebauten ersten Befestigungs- element, einer Verstellvorrichtung mit eng zusammen- gefalteten Schlaufenpaaren von jeweils unterschiedlicher Querausdehnung sowie einem stempelförmigen zweiten Befestigungselement;
- Fign. 3a,b: eine Ausführungsform mit einer Trommelfell-Kopfplatte als erstem Befestigungselement, einer Verstellvorrichtung, die im Ursprungszustand ziehharmonikaförmig auseinander gezogen ist und ohne weitere Schaftteile das längliche Verbindungselement bildet, sowie einer geschlitzten Glocke als zweitem Befestigungselement;
- Fign. 4a,b: eine Ausführungsform mit einem klammerförmigen ersten Befestigungselement, einer Verstellvorrichtung mit ziehharmonikaförmig auseinander gezogenen Schlaufenpaaren von jeweils unterschiedlicher Querausdehnung sowie einem kolbenförmigen zweiten Befestigungselement; und
- Fign. 5a,b: eine Ausführungsform wie in Fig. 4a, jedoch mit Schlaufenpaaren von jeweils gleicher Querausdehnung sowie mit einem Kugelgelenk im Verbindungselement.

Die in den Figuren der Zeichnung schematisch dargestellten fünf - im Detail unterschiedlich gestalteten - Ausführungsformen der erfindungsgemäßen **Gehörknöchelchenprothese 10; 20; 30; 40; 50** weisen am einen Ende jeweils ein **erstes Befestigungselement 11; 21** auf, welches der mechanischen Verbindung der Prothese mit dem Trommelfell oder einem Glied der Gehörknöchelchenkette dient. Am anderen Ende der Gehörknöchelchenprothese 10; 20; 30; 40; 50 sitzt jeweils ein **zweites Befestigungselement 12; 22; 32** zur mechanischen Verbindung der Prothese mit einem weiteren Glied oder Teilen eines Gliedes der Gehörknöchelchenkette oder zum direkten Eintauchen ins Innenohr. Dazwischen ist ein die beiden Befestigungselemente 11; 21 bzw. 12; 22; 32 Schall leitend miteinander verbindendes, längliches **Verbindungselement 13; 23; 33; 43; 53 angeordnet.**

Das Verbindungselement 13; 23; 33; 43; 53 umfasst jeweils eine **Verstellvorrichtung 14; 24; 34; 44; 54** zur Einstellung der axialen Länge einer individuellen Gehörknöchelchenprothese 10; 20; 30; 40; 50 in Achsrichtung des länglichen Verbindungselements 13; 23; 33; 43; 53, wobei jeweils das erste Befestigungselement 11; 21 mit einem Ende und das zweite Befestigungselement 12; 22; 32 mit dem axial entgegen gesetzten anderen Ende des Verbindungselements 13; 23; 33; 43; 53 mechanisch starr verbunden ist.

Erfindungsgemäß umfasst die Verstellvorrichtung 14; 24; 34; 44; 54 mindestens zwei symmetrisch zur **Längsachse a** des Verbindungselements 13; 23; 33; 43; 53 verlaufende, in Achsrichtung streckbare und/oder stauchbare Teilstränge, die zur Festlegung der axialen Länge der Gehörknöchelchenprothese 10; 20; 30; 40; 50 dauerhaft bleibend plastisch verformbar und vor dem Verformen quer zur Längsachse a in mehreren **Schlaufen 15a',15b',15c', 15a",15b",15c"; 25a',25b',25c', 25a",25b",25c"; 35a',35b',35c', 35a",35b",35c"; 45a',45b',45c', 45a",45b",45c"** gefaltet sind.

Bei allen in der Zeichnung gezeigten Ausführungsformen sind quer zur Längsachse a verlaufende **Verbindungsstege 16a,16b** vorgesehen, welche jeweils eine Schlaufe 15a',15b',15c'; 25a',25b',25c'; 35a',35b',35c'; 45a',45b',45c' eines Teilstrangs mit einer Schlaufe 15a",15b",15c"; 25a",25b",25c"; 35a",35b",35c"; 45a",45b",45c" eines parallelen eines Teilstrangs verbinden. Außerdem umfasst jeder Teilstrang genau drei Schlaufen 15a',15b',15c' bzw. 15a",15b",15c"; 25a',25b',25c' bzw. 25a",25b",25c"; 35a',35b',35c' bzw. 35a",35b",35c"; 45a',45b',45c' bzw. 45a",45b",45c", was sich in der Praxis als günstig herausgestellt hat. Bei anderen - in der Zeichnung nicht dargestellten - Ausführungsformen der Erfindung können aber auch weniger oder mehr Schlaufen vorgesehen sein. Die Schlaufen 15a',15b',15c', 15a",15b",15c"; 25a',25b',25c', 25a",25b",25c"; 35a',35b',35c', 35a",35b",35c"; 45a',45b',45c', 45a",45b",45c" der Teilstränge der Verstellvorrichtung 14; 24; 34; 44; 54 können Schlangenförmig, Mäanderförmig bzw. Ziehharmonikaartig gefaltet sein.

Sämtliche in der Zeichnung dargestellten Ausführungsformen der Erfindung stimmen auch darin überein, dass an beiden axialen Enden der Verstellvorrichtung 14; 24; 34; 44; 54 - vorzugsweise mit axialem Abstand zum ersten Befestigungselement 11; 21 bzw. zum zweiten Befestigungselement 12; 22; 32 - jeweils eine **Eingriffseinrichtung 17a,17b** vorgesehen ist, an welcher mit einem Bedingungsinstrument, beispielsweise einer Pinzette oder Zange, jeweils ein Kraft- oder Formschluss hergestellt werden kann, um die Verstellvorrichtung 14; 24; 34; 44; 54 durch Krafteinwirkung in Richtung der Längsachse a des Verbindungselements 13; 23; 33; 43; 53 auseinander zu ziehen oder zu stauchen.

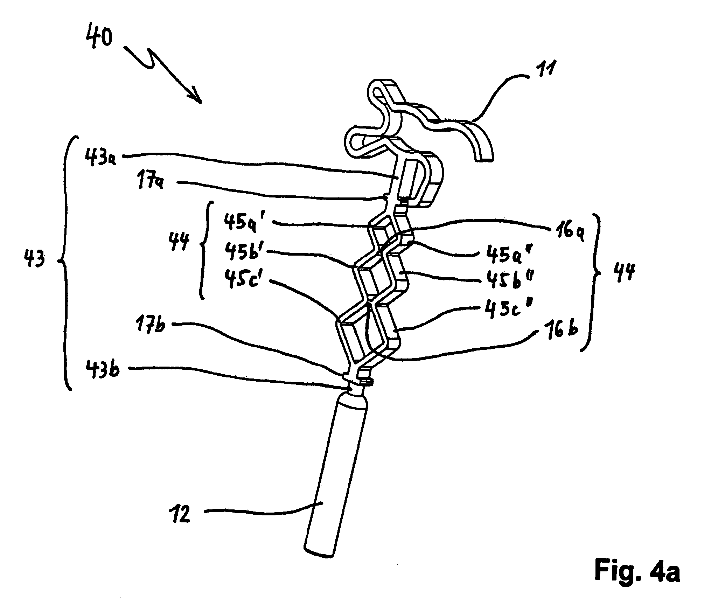

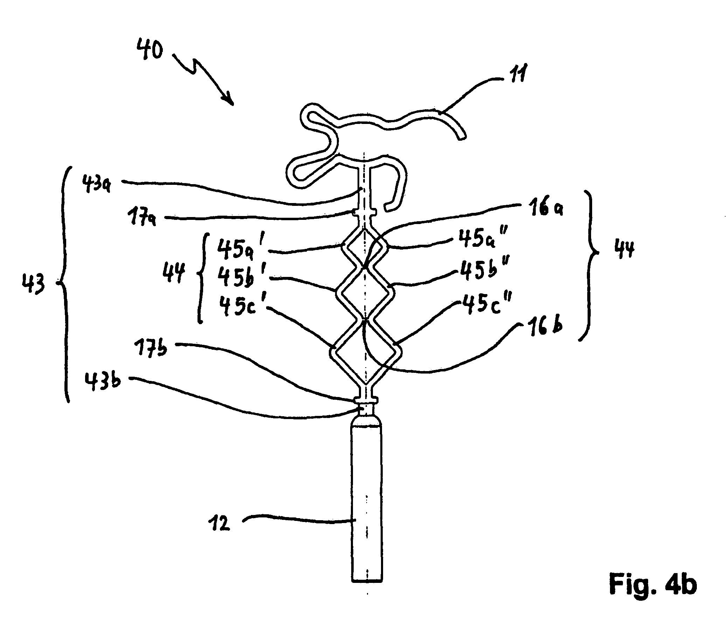

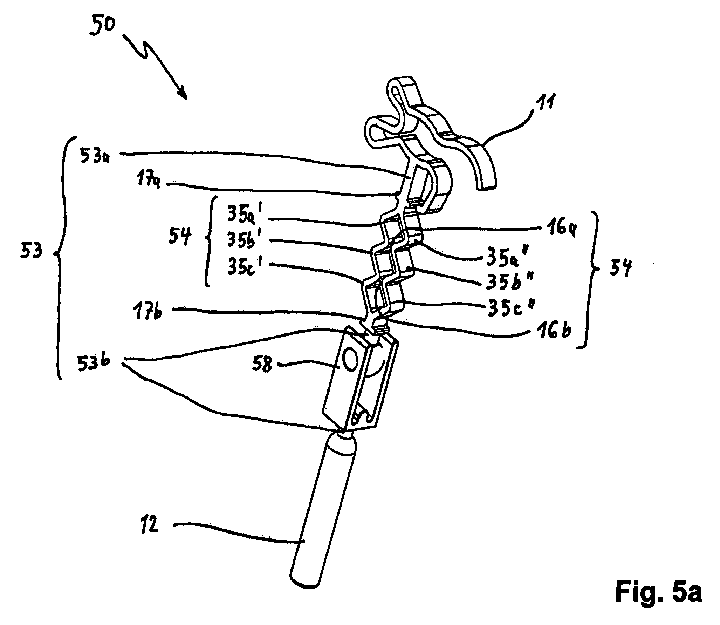

Die in den Figuren 1a-b dargestellte Ausführungsform weist ein erstes Befestigungselement 11 in Form einer Klammer auf, die beispielsweise auf den Ambossfortsatz oder auch auf ein anderes Glied der Gehörknöchelchenkette aufgeclipst werden kann. Das zweite Befestigungselement 12 an dem der Klammer entgegen gesetzten Ende ist in diesem Ausführungsbeispiel als Kolben zur direkten Ankopplung der Gehörknöchelchenprothese 10 an das Innenohr ausgebildet. Die gleiche Ausbildung der Befestigungselemente zeigen auch die Ausführungsformen der Gehörknöchelchenprothesen 40 und 50 gemäß den Figuren 4a-b und 5a-b.

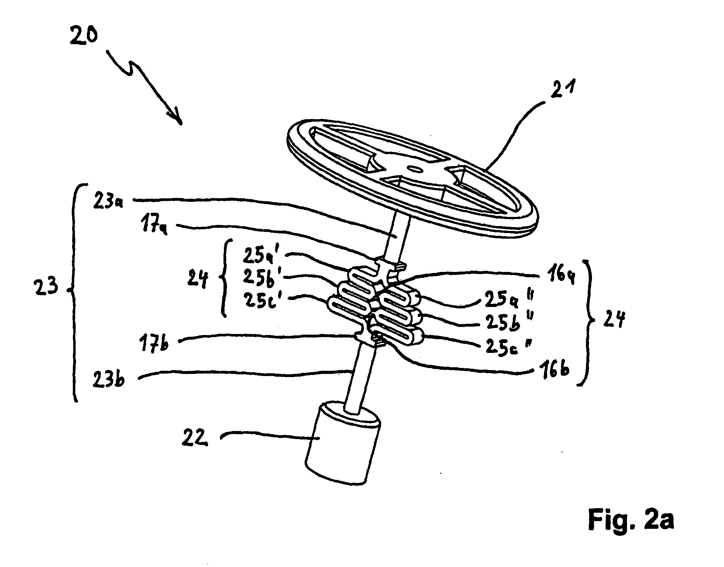

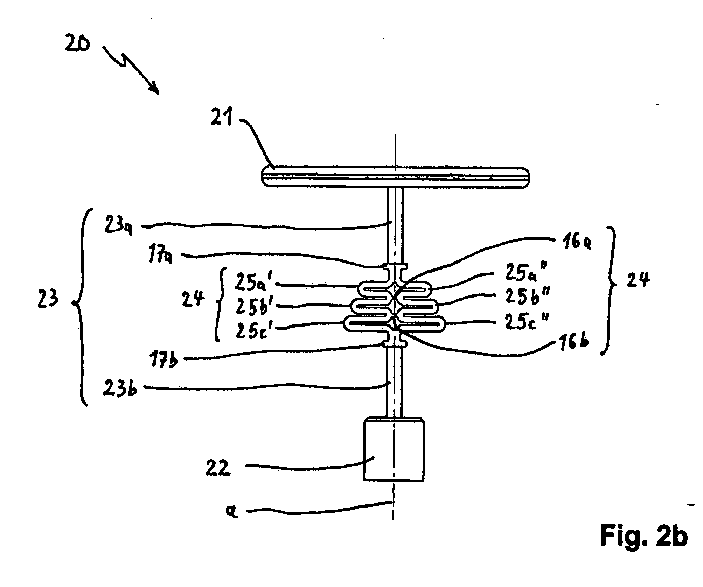

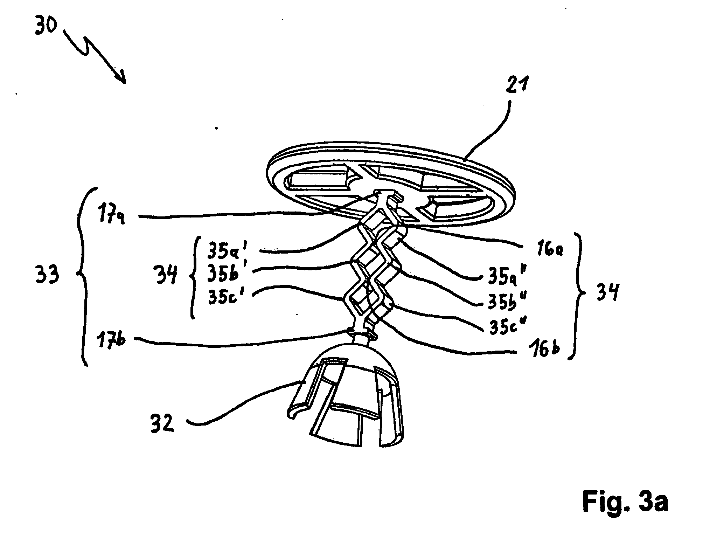

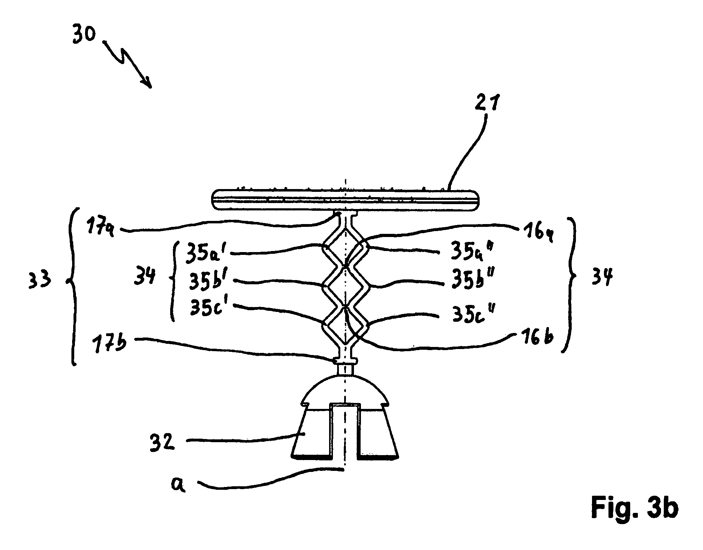

Bei den Ausführungsformen nach den Figuren 2a-b und 3a-b hingegen ist das erste Befestigungselement 21 jeweils in Form einer Kopfplatte zur Anlage am Trommelfell ausgebildet. Das zweite Befestigungselement 22 an dem der Kopfplatte entgegen gesetzten Ende ist im Ausführungsbeispiel der Gehörknöchelchenprothese 20 nach den Figuren 2a-b stempelförmig zur Auflage auf der Steigbügelfußplatte geformt, während das zweite Befestigungselement 32 im Ausführungsbeispiel der Gehörknöchelchenprothese 30 nach den Figuren 3a-b als geschlitzte Glocke gestaltet ist und vorzugsweise zur Befestigung der Gehörknöchelchenprothese 30 auf dem Steigbügel dient.

Bei in der Zeichnung nicht dargestellten Ausführungsformen der erfindungsgemäßen Gehörknöchelchenprothese können die Befestigungselemente auch anders gestaltet sein, etwa als Hülse, Schlinge oder Haken. Auch kann beispielsweise das zweite Befestigungselement in Form einer Klammer ausgebildet sein.

In der Regel wird das längliche Verbindungselement 13; 23; 43; 53 als zweigeteilter Schaft gestaltet sein, wie in den Ausführungsbeispielen der Figuren 1a-b, 2a-b, 4a-b und 5a-b gezeigt. Die Verstellvorrichtung 14; 24; 44; 54 ist hier jeweils in den Schaft integriert ist und an beiden axialen Enden der Verstellvorrichtung 14; 24; 44; 54 ist ein **Verbindungsstück 13a; 23a; 43a; 53a bzw. 13b; 23b; 43b; 53b** zum ersten Befestigungselement 11; 21 bzw. zum zweiten Befestigungselement 12; 22 angeordnet. Bei der Ausführungsform nach den Figuren 3a-b hingegen bildet die Verstellvorrichtung 34 selbst das längliche Verbindungselement 33.

Wie die Ausführungsformen der Figuren 1a-b und 2a-b zeigen, können die Schlaufen 15a',15b',15c', 15a",15b",15c"; 25a',25b',25c', 25a", 25b",25c" der Verstellvorrichtung 14; 24 im Grundzustand der Gehörknöchelchenprothese 10; 20 eng zusammengefaltet sein. Sie werden dann zur Einstellung einer gewünschten axialen Länge der Prothese in Richtung der Längsachse a des länglichen Verbindungselements 13; 23 von einem Operateur auseinander gezogen werden und verharren nach der Implantation der Prothese ins Mittelohr des Patienten in diesem auseinander gezogenen Zustand dauerhaft plastisch verformt.

Alternativ dazu sind bei den Ausführungsbeispielen der Figuren 3a-b, 4a-b und 5a-b die Schlaufen 35a',35b',35c', 35a",35b",35c"; 45a',45b',45c', 45a",45b",45c" der Verstellvorrichtung 34; 44; 54 im angelieferten Zustand der Gehörknöchelchenprothesen 30; 40; 50 auseinander gezogen und werden zur Einstellung der gewünschten axialen Länge der Prothese in Richtung der Längsachse a des länglichen Verbindungselements 33; 43; 53 zusammengestaucht.

Die Ausführungsformen nach den Figuren 1a-b, 3a-b und 5a-b zeichnen sich weiterhin dadurch aus, dass die Schlaufen 15a',15b',15c', 15a",15b",15c"; 35a',35b',35c', 35a",35b",35c" der Verstellvorrichtung 14; 34; 54 quer zur Längsachse a des länglichen Verbindungselements 13; 33; 53 jeweils die gleiche maximale Ausdehnung aufweisen. Im Gegensatz dazu weisen bei den Ausführungsformen nach den Figuren 2a-b und 4a-b axial gegenüberliegende Schlaufenpaare 25a',25a", 25b',25b", 25c',25c"; 45a',45a", 45b',45b", 45c',45c" der Verstellvorrichtung 24; 44 quer zur Längsachse a des länglichen Verbindungselements 23; 43 unterschiedliche, insbesondere von einem axialen Ende der Verstellvorrichtung 24; 44 zum anderen axialen Ende hin stetig zunehmende oder abnehmende maximale Ausdehnungen auf.

In der Ausführungsform nach den Figuren 5a-b ist in das Verbindungselement 53 ein **Kugelgelenk 58** integriert, um eine gewisse postoperative Flexibilität der Gehörknöchelchenprothese 50 zwischen ihren Verbindungsstellen sicherzustellen.

Die Massenverteilung der einzelnen Teile der erfindungsgemäßen Gehörknöchelchenprothese 10; 20; 30; 40; 50 kann in Abhängigkeit von einem gewünschten, vorgebbaren Frequenzgang der Schallleitung im Mittelohr derart berechnet sein, dass ein individuelles Tuning der Schallleitungs-Eigenschaften ermöglicht wird.

## Patentansprüche

1. Gehörknöchelchenprothese (10; 20; 30; 40; 50), die mindestens ein Glied der menschlichen Gehörknöchelchenkette ersetzt oder überbrückt, wobei die Gehörknöchelchenprothese (10; 20; 30; 40; 50) an einem Ende ein erstes Befestigungselement (11; 21) zur mechanischen Verbindung mit dem Trommelfell oder einem Glied der Gehörknöchelchenkette und an ihrem anderen Ende ein zweites Befestigungselement (12; 22; 32) zur mechanischen Verbindung mit einem weiteren Glied oder Teilen eines Gliedes der Gehörknöchelchenkette oder direkt mit dem Innenohr sowie ein die beiden Befestigungselemente (11; 21 bzw. 12; 22; 32) Schall leitend miteinander verbindendes, längliches Verbindungselement (13; 23; 33; 43; 53) aufweist, welches eine Verstellvorrichtung (14; 24; 34; 44; 54) zur Einstellung der axialen Länge der Gehörknöchelchenprothese (10; 20; 30; 40; 50) in Achsrichtung des länglichen Verbindungselements (13; 23; 33; 43; 53) umfasst, wobei das erste Befestigungselement (11; 21) mit einem Ende und das zweite Befestigungselement (12; 22; 32) mit dem axial entgegen gesetzten anderen Ende des Verbindungselements (13; 23; 33; 43; 53) mechanisch starr verbunden ist,
**dadurch gekennzeichnet,**
**dass** die Verstellvorrichtung (14; 24; 34; 44; 54) mindestens zwei symmetrisch zur Längsachse (a) des Verbindungselements (13; 23; 33; 43; 53) verlaufende, in Achsrichtung streckbare und/oder stauchbare Teilstränge umfasst, die dauerhaft bleibend plastisch verformbar und vor dem Verformen quer zur Längsachse (a) in mehreren Schlaufen (15a',15b',15c', 15a",15b",15c"; 25a',25b',25c', 25a",25b",25c"; 35a',35b',35c', 35a",35b",35c"; 45a',45b',45c', 45a",45b",45c") gefaltet sind.

2. Gehörknöchelchenprothese nach Anspruch 1, **dadurch gekennzeichnet, dass** mindestens ein, vorzugsweise mehrere quer zur Längsachse (a) verlaufende Verbindungsstege (16a,16b), vorgesehen sind, welche jeweils eine Schlaufe (15a',15b',15c'; 25a',25b',25c'; 35a',35b',35c'; 45a',45b',45c') eines Teilstrangs mit einer Schlaufe (15a",15b",15c"; 25a",25b",25c"; 35a",35b",35c"; 45a",45b",45c") eines parallelen eines Teilstrangs verbinden.

3. Gehörknöchelchenprothese nach einem der vorhergehenden Ansprüche, **dadurch gekennzeichnet, dass** jeder Teilstrang mindestens drei Schlaufen (15a',15b',15c' bzw. 15a",15b",15c"; 25a',25b',25c' bzw. 25a",25b",25c"; 35a',35b',35c' bzw. 35a",35b",35c"; 45a',45b',45c' bzw. 45a",45b",45c") umfasst.

4. Gehörknöchelchenprothese nach einem der Ansprüche 1 bis 3, **dadurch gekennzeichnet, dass** die Schlaufen (15a',15b',15c', 15a",15b",15c"; 25a',25b',25c', 25a",25b",25c") der Verstellvorrichtung (14; 24) im angelieferten Zustand der Gehörknöchelchenprothese (10; 20) eng zusammengefaltet sind, zur Einstellung einer gewünschten axialen Länge der Prothese in Richtung der Längsachse (a) des länglichen Verbindungselements (13; 23) von einem Operateur auseinander gezogen werden können und nach der Implantation der Prothese ins Mittelohr des Patienten in diesem auseinander gezogenen Zustand plastisch verformt verharren.

5. Gehörknöchelchenprothese nach einem der Ansprüche 1 bis 3, **dadurch gekennzeichnet, dass** die Schlaufen (35a',35b',35c', 35a",35b",35c"; 45a',45b',45c', 45a",45b",45c") der Verstellvorrichtung (34; 44; 54) im angelieferten Zustand der Gehörknöchelchenprothese (30; 40; 50) auseinander gezogen sind, zur Einstellung einer gewünschten axialen Länge der Prothese in Richtung der Längsachse (a) des länglichen Verbindungselements (33; 43; 53) von einem Operateur zusammen gestaucht werden können und nach der Implantation der Prothese ins Mittelohr des Patienten in diesem zusammengestauchten Zustand plastisch verformt verharren.

6. Gehörknöchelchenprothese nach einem der vorhergehenden Ansprüche, **dadurch gekennzeichnet, dass** die Schlaufen (15a',15b',15c', 15a",15b",15c"; 25a',25b',25c', 25a",25b",25c"; 35a',35b',35c', 35a",35b",35c"; 45a',45b',45c', 45a",45b",45c") der Teilstränge der Verstellvorrichtung (14; 24; 34; 44; 54) Schlangenförmig, Mäanderförmig bzw. Ziehharmonika-artig gefaltet sind.

7. Gehörknöchelchenprothese nach einem der Ansprüche 1 bis 6, **dadurch gekennzeichnet, dass** die Schlaufen (15a',15b',15c', 15a",15b",15c"; 35a',35b',35c', 35a",35b",35c") der Verstellvorrichtung (14; 34; 54) quer zur Längsachse (a) des länglichen Verbindungselements (13; 33; 53) jeweils die gleiche maximale Ausdehnung aufweisen.

8. Gehörknöchelchenprothese nach einem der Ansprüche 1 bis 6, **dadurch gekennzeichnet, dass** axial gegenüberliegende Schlaufenpaare (25a',25a", 25b',25b", 25c',25c"; 45a',45a", 45b',45b", 45c',45c") der Verstellvorrichtung (24; 44) quer zur Längsachse (a) des länglichen Verbindungselements (23; 43) unterschiedliche, insbesondere von einem axialen Ende der Verstellvorrichtung (24; 44) zum anderen axialen Ende hin stetig zunehmende oder abnehmende maximale Ausdehnungen aufweisen.

9. Gehörknöchelchenprothese nach einem der Ansprüche 1 bis 8, **dadurch gekennzeichnet, dass** die Verstellvorrichtung (34) das längliche Verbindungselement (33) bildet.

10. Gehörknöchelchenprothese nach einem der Ansprüche 1 bis 8, **dadurch gekennzeichnet, dass** das längliche Verbindungselement (13; 23; 43; 53) als Schaft gestaltet ist.

11. Gehörknöchelchenprothese nach Anspruch 10, **dadurch gekennzeichnet, dass** die Verstellvorrichtung (14; 24; 44; 54) in den Schaft integriert ist und dass an beiden axialen Enden der Verstellvorrichtung (14; 24; 44; 54) ein Verbindungsstück (13a; 23a; 43a; 53a bzw. 13b; 23b; 43b; 53b) zum ersten Befestigungselement (11; 21) bzw. zum zweiten Befestigungselement (12; 22) angeordnet ist.

12. Gehörknöchelchenprothese nach einem der vorhergehenden Ansprüche, **dadurch gekennzeichnet, dass** an beiden axialen Enden der Verstellvorrichtung (14; 24; 34; 44; 54), vorzugsweise mit axialem Abstand zum ersten Befestigungselement (11; 21) bzw. zum zweiten Befestigungselement (12; 22; 32), jeweils eine Eingriffseinrichtung (17a,17b) vorgesehen ist, an welcher mit einem Bedingungsinstrument, beispielsweise einer Pinzette oder Zange, jeweils ein Kraft- oder Formschluss hergestellt werden kann, um die Verstellvorrichtung (14; 24; 34; 44; 54) durch Krafteinwirkung in Richtung der Längsachse (a) des Verbindungselements (13; 23; 33; 43; 53) auseinander zu ziehen oder zu stauchen.

13. Gehörknöchelchenprothese nach einem der vorhergehenden Ansprüche, **dadurch gekennzeichnet, dass** das Verbindungselement (53) mindestens ein Gelenk, vorzugsweise ein Kugelgelenk (58), insbesondere eine axial verlaufende Kugelgelenk-Kette aufweist.

14. Gehörknöchelchenprothese nach einem der vorhergehenden Ansprüche, **dadurch gekennzeichnet, dass** mindestens eines der beiden Befestigungselemente (11; 21 bzw. 12; 22; 32) als Platte, insbesondere als Kopfplatte zur Anlage am Trommelfell, als Hülse, als Schlinge, als vorzugsweise rund gebogener, teilweise offener Haken, als geschlossene Glocke, als einfach oder mehrfach geschlitzte Glocke oder als Clip zur mechanischen Verbindung mit einem Glied der Gehörknöchelchenkette ausgebildet ist.

15. Gehörknöchelchenprothese nach einem der vorhergehenden Ansprüche, **dadurch gekennzeichnet, dass** Teile der Gehörknöchelchenprothese (10; 20; 30; 40; 50) aus einem Material mit Formgedächtnis (=memory effect), insbesondere aus Nitinol hergestellt sind.

## Claims

1. Ossicular prosthesis (10; 20; 30; 40; 50) replacing or overbridging at least one member of the human ossicular chain, the ossicular prosthesis (10; 20; 30; 40; 50) having at one end a first attachment element (11; 21) for mechanical connection to the tympanic membrane or to a member of the ossicular chain and at its other end a second attachment element (12; 22; 32) for mechanical connection to a further member, or parts of a member, of the ossicular chain or directly to the inner ear, and an elongate connecting element (13; 23; 33; 43; 53) interconnecting and acoustically coupling the two attachment elements (11; 21 and 12; 22; 32 respectively) which comprises an adjusting device (14; 24; 34; 44; 54) for adjusting the axial length of the ossicular prosthesis (10; 20; 30; 40; 50) in the axial direction of the elongate connecting element (13; 23; 33; 43; 53), the first attachment element (11; 21) being mechanically rigidly connected to one end, and the second attachment element (12; 22; 32), mechanically rigidly connected to the other, axially opposite, end, of the connecting element (13; 23; 33; 43; 53),
**characterized in that**
the adjusting device (14; 24; 34; 44; 54) comprises at least two partial legs extending symmetrically with respect to the longitudinal axis (a) of the connecting element (13; 23; 33; 43; 53) that are extensible and/or compressible in the axial direction and are permanently plastically deformable and before being deformed are folded at right angles to the longitudinal axis (a) in a number of loops (15a', 15b', 15c', 15a", 15b", 15c"; 25a', 25b', 25c', 25a", 25b", 25c"; 35a', 35b', 35c', 35a", 35b", 35c"; 45a', 45b', 45c', 45a", 45b", 45c").

2. Ossicular prosthesis according to Claim 1, **characterized in that** at least one, and preferably more than one, bridging webs (16a, 16b) extending at right angles to the longitudinal axis (a) are provided, each connecting a loop (15a', 15b', 15c'; 25a', 25b', 25c'; 35a', 35b', 35c'; 45a', 45b', 45c') of one partial leg to a loop (15a", 15b", 15c"; 25a", 25b", 25c"; 35a", 35b", 35c"; 45a", 45b", 45c") of a parallel partial leg.

3. Ossicular prosthesis according to either of the preceding claims, **characterized in that** each partial leg comprises three loops (15a', 15b', 15c' and 15a", 15b", 15c" respectively; 25a', 25b', 25c' and 25a", 25b", 25c" respectively; 35a', 35b', 35c' and 35a", 35b", 35c" respectively; 45a', 45b', 45c' and 45a", 45b", 45c" respectively).

4. Ossicular prosthesis according to any one of Claims 1 to 3, **characterized in that** the loops (15a', 15b', 15c', 15a", 15b", 15c"; 25a', 25b', 25c', 25a", 25b", 25c") of the adjusting device (14; 24) are packed close together in the as-supplied condition of the ossicular prosthesis (10; 20) but can be pulled apart by an operator in the direction of the longitudinal axis (a) of the elongate connecting element (13; 23) for adjustment to a desired axial length of the prosthesis, and remain plastically deformed **in that** extended condition after implantation of the prosthesis into the patient's middle ear.

5. Ossicular prosthesis according to any one of Claims 1 to 3, **characterized in that** the loops (35a', 35b', 35c', 35a", 35b", 35c"; 45a', 45b', 45c', 45a", 45b", 45c") of the adjusting device (34; 44; 54) are drawn apart in the as-supplied condition of the ossicular prosthesis (30; 40; 50) but can be squeezed together by an operator in the direction of the longitudinal axis (a) of the elongate connecting element (33; 43; 53) for adjustment to a desired axial length of the prosthesis, and remain plastically deformed **in that** compressed condition after implantation of the prosthesis into the patient's middle ear.

6. Ossicular prosthesis according to any one of the preceding claims, **characterized in that** the loops (15a', 15b', 15c', 15a", 15b", 15c"; 25a', 25b', 25c', 25a", 25b", 25c"; 35a', 35b', 35c', 35a", 35b", 35c"; 45a', 45b', 45c', 45a", 45b", 45c") of the partial legs of the adjusting device (14; 24; 34; 44; 54) are folded in meanders or in serpentine or concertina fashion.

7. Ossicular prosthesis according to any one of Claims 1 to 6, **characterized in that** each of the loops (15a', 15b', 15c', 15a", 15b", 15c"; 35a', 35b', 35c', 35a", 35b", 35c") of the adjusting device (14; 34; 54) has the same maximum extent at right angles to the longitudinal axis (a) of the elongate connecting element (13; 33; 53).

8. Ossicular prosthesis according to any one of Claims 1 to 6, **characterized in that** pairs of axially opposite loops (25a', 25a", 25b', 25b", 25c', 25c"; 45a', 45a", 45b', 45b", 45c', 45c") of the adjusting device (24; 44) have different maximum extent at right angles to the longitudinal axis (a) of the elongate connecting element (23; 43), in particular a maximum extent that constantly increases or decreases from one axial end of the adjusting device (24; 44) towards the other.

9. Ossicular prosthesis according to any one of Claims 1 to 8, **characterized in that** the adjusting device (34) forms the elongate connecting element (33).

10. Ossicular prosthesis according to any one of Claims 1 to 8, **characterized in that** the elongate connecting element (13; 23; 43; 53) is designed as a shank.

11. Ossicular prosthesis according to Claim 10, **characterized in that** the adjusting device (14; 24; 44; 54) is integral with the shank and **in that** connecting pieces (13a; 23a; 43a; 53a and 13b; 23b; 43b; 53b respectively) joining it to the first attachment element (11; 21) and second attachment element (12; 22) respectively are arranged at the two axial ends of the adjusting device (14; 24; 44; 54).

12. Ossicular prosthesis according to any one of the preceding claims, **characterized in that** an operational aid (17a, 17b) is provided at each axial end of the adjusting device (14; 24; 34; 44; 54), preferably at an axial distance from the first attachment element (11; 21) and second attachment element (12; 22; 32) respectively, at which a positive or non-positive connection can be made with a [manipulating] instrument, e.g. forceps or tweezers, to extend or compress the adjusting device (14; 24; 34; 44; 54) by applying force in the direction of the longitudinal axis (a) of the connecting element (13; 23; 33; 43; 53).

13. Ossicular prosthesis according to any one of the preceding claims, **characterized in that** the connecting element (53) has at least one articulated joint, preferably a ball joint (58), and in particular an axially extending ball-joint chain.

14. Ossicular prosthesis according to any one of the preceding claims, **characterized in that** at least one of the two attachment elements (11; 21 or 12; 22; 32) is configured as a plate, in particular as a top plate for contact with the tympanic membrane, or as a sleeve or a loop, or a preferably rounded, partly open, hook, or a closed cup, a cup with one or more slots, or a clip for mechanical connection to a member of the ossicular chain.

15. Ossicular prosthesis according to any one of the preceding claims, **characterized in that** parts of the ossicular prosthesis (10; 20; 30; 40; 50) are made of shape-memory material, in particular nitinol.

## Revendications

1. Prothèse des osselets de l'oreille (10 ; 20 ; 30 ; 40 ; 50), qui remplace ou ponte un segment de la chaîne d'osselets de l'oreille humains, dans laquelle la prothèse d'osselets de l'oreille (10 ; 20 ; 30 ; 40 ; 50) présente, à une extrémité, un premier élément de fixation (11 ; 21) pour la liaison mécanique avec le tympan ou un segment de la chaîne d'osselets de l'oreille et, à son autre extrémité, un deuxième élément de fixation (12 ; 22 ; 32) pour la liaison mécanique avec un autre segment ou des parties d'un segment de la chaîne d'osselets de l'oreille ou directement avec l'oreille interne ainsi qu'un élément de liaison (13 ; 23 ; 33 ; 43 ; 53) allongé raccordant l'un à l'autre les deux éléments de fixation (11 ; 21 ou 12 ; 22 ; 32) de manière à conduire le son, lequel élément de liaison comprend un dispositif de réglage (14 ; 24 ; 34 ; 44 ; 54) pour régler la longueur axiale de la prothèse d'osselets de l'oreille (10 ; 20 ; 30 ; 40 ; 50) dans la direction axiale de l'élément de liaison allongé (13 ; 23 ; 33 ; 43 ; 53), dans laquelle le premier élément de fixation (11 ; 21) est relié de manière mécaniquement rigide à une extrémité et le deuxième élément de fixation (12 ; 22 ; 32) à l'autre extrémité axialement opposée de l'élément de liaison (13 ; 23 ; 33 ; 43 ; 53),
**caractérisée en ce que**
le dispositif de réglage (14 ; 24 ; 34 ; 44 ; 54) comprend au moins deux branches partielles s'étendant symétriquement à l'axe longitudinal (a) de l'élément de liaison (13 ; 23 ; 33 ; 43 ; 53) et étirable et/ou refoulable dans la direction axiale, lesquelles branches peuvent subir une déformation plastique permanente durable et sont pliées avant la déformation transversalement à l'axe longitudinal (a) en plusieurs boucles (15a', 15b',15c',15a",15b",15c" ; 25a',25b',25c',25a", 25b",25c" ; 35a',35b',35c',35a",35b",35c" ; 45a', 45b',45c',45a",45b",45c").

2. Prothèse d'osselets de l'oreille selon la revendication 1, **caractérisée en ce qu'**il est prévu au moins un, de préférence plusieurs âmes de liaison (16a,16b) s'étendant transversalement à l'axe longitudinal (a), qui relient respectivement une boucle (15a',15b',15c' ; 25a',25b',25c' ; 35a',35b',35c' ; 45a', 45b',45c') d'une branche partielle à une boucle (15a",15b",15c" ; 25a", 25b",25c" ; 35a",35b",35c" ; 45a",45b",45c") d'une branche partielle parallèle.

3. Prothèse d'osselets de l'oreille selon l'une quelconque des revendications précédentes, **caractérisée en ce que** chaque branche partielle comprend au moins trois boucles (15a', 15b',15c' ou 15a",15b",15c" ; 25a',25b',25c' ou 25a",25b", 25c" ; 35a',35b',35c' ou 35a",35b",35c" ; 45a', 45b',45c' ou 45a",45b",45c").

4. Prothèse d'osselets de l'oreille selon l'une quelconque des revendications 1 à 3, **caractérisée en ce que** les boucles (15a',15b',15c',15a",15b", 15c" ; 25a',25b',25c',25a",25b",25c") du dispositif de réglage (14 ; 24) sont étroitement repliées à l'état livré de la prothèse d'osselets de l'oreille (10 ; 20), peuvent être écartées l'une de l'autre par un opérateur pour régler une longueur axiale souhaitée de la prothèse dans la direction de l'axe longitudinal (a) de l'élément de liaison allongé (13 ; 23) et rester déformées à l'état plastique dans cet écartement mutuel après l'implantation de la prothèse dans l'oreille moyenne du patient.

5. Prothèse d'osselets de l'oreille selon l'une quelconque des revendications 1 à 3, **caractérisée en ce que** les boucles (35a',35b',35c',35a",35b", 35c" ; 45a',45b',45c',45a",45b",45c") du dispositif de réglage (34 ; 44 ; 54) sont écartées l'une de l'autre à l'état livré de la prothèse d'osselets de l'oreille (30 ; 40 ; 50), peuvent être refoulées conjointement par un opérateur pour régler une longueur axiale souhaitée de la prothèse dans la direction de l'axe longitudinal (a) de l'élément de liaison allongé (33 ; 43 ; 53) et rester déformées à l'état plastique dans ce refoulement conjoint après l'implantation de la prothèse dans l'oreille moyenne du patient.

6. Prothèse d'osselets de l'oreille selon l'une quelconque des revendications précédentes, **caractérisée en ce que** les boucles (15a', 15b',15c',15a",15b",15c" ; 25a',25b',25c',25a", 25b",25c" ; 35a',35b',35c',35a",35b",35c", 45a', 45b',45c', 45a",45b",45c") des branches partielles du dispositif de réglage (14 ; 24 ; 34 ; 44 ; 54) sont repliées en formes de serpentins, en forme de méandres ou en accordéon.

7. Prothèse d'osselets de l'oreille selon l'une quelconque des revendications 1 à 6, **caractérisée en ce que** les boucles (15a',15b',15c',15a", 15b",15c" ; 35a',35b',35c',35a",35b",35c" du dispositif de réglage (14 ; 34 ; 54) présentent respectivement la même extension maximale transversalement à l'axe longitudinal (a) de l'élément de liaison allongé (13 ; 33 ; 53).

8. Prothèse d'osselets de l'oreille selon l'une quelconque des revendications 1 à 6, **caractérisée en ce que** la paire de boucles axialement opposées (25a',25b',25c',25a",25b",25c" ; 45a', 45b',45c', 45a",45b",45c") présente transversalement à l'axe longitudinal (a) de l'élément de liaison allongé (23 ; 44) différentes extensions maximales de plus en plus grandes ou de plus en plus petites, en particulier d'une extrémité axiale du dispositif de réglage à l'autre extrémité axiale.

9. Prothèse d'osselets de l'oreille selon l'une quelconque des revendications 1 à 8, **caractérisée en ce que** le dispositif de réglage (34) forme l'élément de liaison allongé (33).

10. Prothèse d'osselets de l'oreille selon l'une quelconque des revendications 1 à 8, **caractérisée en ce que** l'élément de liaison allongé (13 ; 23 ; 43 ; 53) en conformé en tige.

11. Prothèse d'osselets de l'oreille selon la revendication 10, **caractérisée en ce que** le dispositif de réglage (14 ; 24 ; 44 ; 54) est intégré à la tige et **en ce que** l'on agence sur les deux extrémités axiales du dispositif de réglage (14 ; 24 ; 44 ; 54) une pièce de liaison (13a ; 23a ; 43a ; 53a ou 13b ; 23b ; 43b ; 53b) pour le premier élément de fixation (11 ; 21) ou pour le deuxième élément de fixation (12 ; 22).

12. Prothèse d'osselets de l'oreille selon l'une quelconque des revendications précédentes, **caractérisée en ce qu'**il est prévu sur les deux extrémités axiales du dispositif de réglage (14 ; 24 ; 34 ; 44 ; 54), de préférence à distance axiale du premier élément de fixation (11 ; 21) ou du deuxième élément de fixation (12 ; 22 ; 32), respectivement un dispositif de préhension (17a,17b), sur lequel, à l'aide d'un instrument de manipulation, par exemple une petite pince, on peut respectivement assurer un ajustement à force ou une adaptation de forme pour écarter ou refouler le dispositif de réglage (14 ; 24 ; 34 ; 44 ; 54) par application d'une force dans la direction de l'axe longitudinal (a) de l'élément de liaison (13 ; 23 ; 33 ; 43 ; 53).

13. Prothèse d'osselets de l'oreille selon l'une quelconque des revendications précédentes, **caractérisée en ce que** l'élément de liaison (53) présente au moins une articulation, de préférence une articulation à rotule (58), en particulier une chaîne d'articulations à rotule s'étendant axialement.

14. Prothèse d'osselets de l'oreille selon l'une quelconque des revendications précédentes, **caractérisée en ce qu'**au moins l'un des deux éléments de fixation (11 ; 21 ou 12 ; 22 ; 32) se présente sous la forme d'une plaque, en particulier d'une plaque de tête pour appuyer sur le tympan, d'une douille, d'une boucle, d'un crochet de préférence incurvé en rond et en partie ouvert, d'une cloche fermée, d'une cloche incisée de manière unique ou multiple ou d'une pince pour assurer une liaison mécanique avec un segment de la chaîne d'osselets de l'oreille.

15. Prothèse d'osselets de l'oreille selon l'une quelconque des revendications précédentes, **caractérisée en ce que** des parties de la prothèse d'osselets de l'oreille (10 ; 20 ; 30 ; 40 ; 50) sont fabriquées dans un matériau à mémoire de forme, en particulier de nitinol.
